# EUROPEAN PATENT SPECIFICATION

(11) **EP 2 805 675 B1**
(45) Date of publication and mention of the grant of the patent: **08.04.2015**
(21) Application number: 13168675.0
(22) Date of filing: 22.05.2013
(51) Int. Cl.: A61B 5/15, A61B 5/153

(54) **Blood sampling device for sampling from a fluid line**
Blutprobenentnahmevorrichtung zur Probenentnahme aus einer Fluidleitung
Dispositif de prélèvement sanguin pour le prélèvement d'une ligne de fluide

(43) Date of publication of application: 26.11.2014
(73) Proprietor: IN. Medica, d.o.o., 8310 Sentjernej (SI)
(72) Inventor: Simoncic, Alojz, 8310 SENTJERNEJ (SI)
(74) Representative: Ivancic, Bojan

(56) References cited:
- EP-A1- 0 376 603
- EP-A2- 0 301 913
- EP-B1- 0 575 916
- WO-A1-89/01616
- WO-A1-2012/146735
- GB-A- 2 187 234

## Description

The present invention refers to a blood sampling device comprising a piston-cylinder arrangement as an intermediate storage for diluted blood, said piston-cylinder arrangement comprises a cylindrical housing cooperating with a through-flow channel and being closed by means of a rotating handle, and a piston moving within said cylindrical housing and connected to said handle.

The aforementioned device has been disclosed in the patent documents No. WO89/01616 A1, GB 2 187 234 A, EP 0 575 916 B1, WO 2012/146735 A1 and EP 0 376 603 A1. It is the problem with the known devices to be rather difficult to use, particularly with different sampling requirements, and are both space and money consuming.

It is the object of the present invention to create a blood sampling device of the aforementioned kind, which remedies drawbacks of known dosing devices.

The object as set above is solved by characteristics set forth in a characterising clause of the claim 1. Particularities of the invention are further disclosed in subclaims.

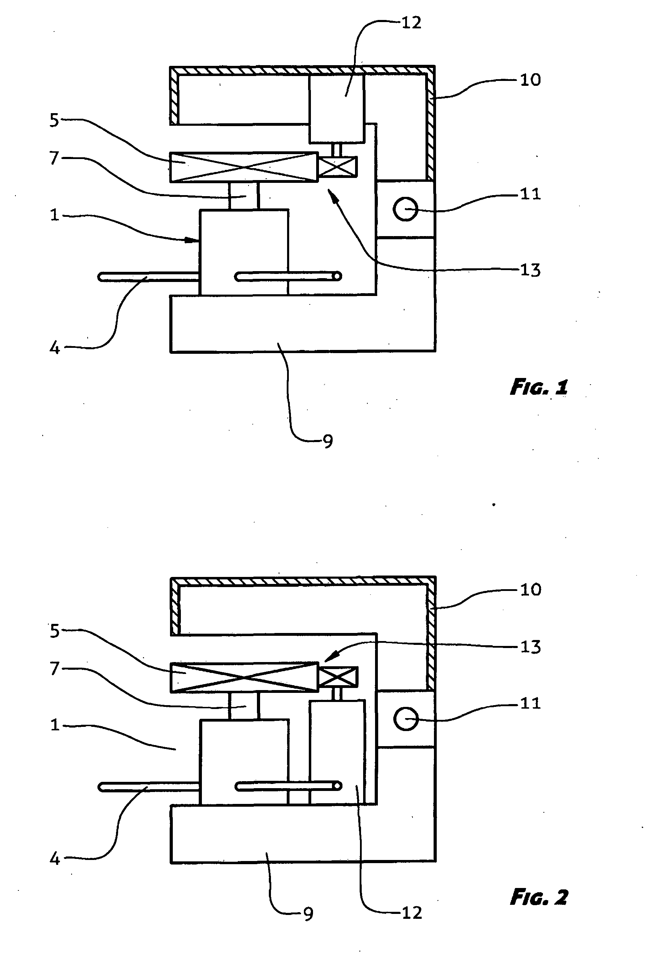

The invention is further described in detail by way of non-limiting embodiments, and with a reference to the accompanying drawings, where
- Fig. 1: shows a schematic view of a blood sampling device according to the invention,
- Fig. 2: shows another embodiment of the device of Fig. 1, and

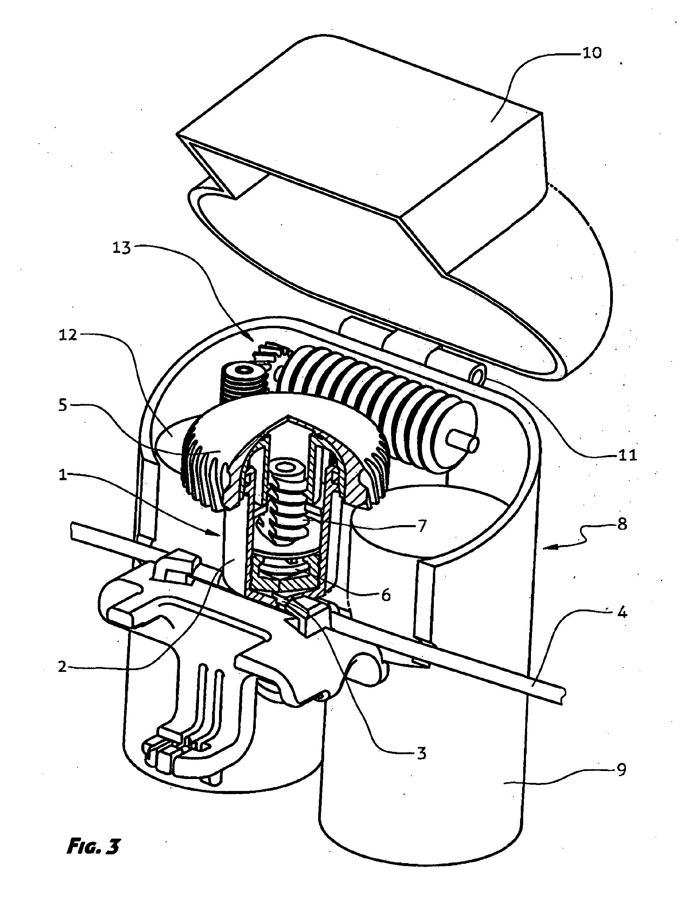

- Fig. 3: shows a three-dimensional view of an embodiment of the device according to the invention.

A blood sampling device according to the invention comprises a piston-cylinder arrangement 1 serving as an intermediate storage for a diluted blood. Said arrangement 1 comprises a housing 2 which cooperates via a tubular protrusion 3 with a conduit 4 and is closed by means of a rotating handle 5. A movable piston 6 is arranged inside said housing 2 which is connected by means of a piston rod 7 to said rotating handle 5. Said conduit 4 is connected at the first end thereof with a patient, by means of a syringe for instance, and is connected at the second end thereof with a source of the infusion liquid, a physiological dilution for instance. Furthermore, a device for closing and/or redirecting the flow of the liquids known per se is arranged in said conduit 4 in the area between said arrangement 1 and said syringe and the patient, respectively.

Further, said blood sampling device according to the invention comprises a holder 8 formed of at least one fixed section 9 and at least one moveable, preferably swivelling section 10 being mutually interconnected by means of a swivel joint 11. Said swivelling section 10 is formed in a manner that in the swivelled position, i.e. in the operating position for a blood sampling, the free end thereof is located in the area directly above said rotating handle 5 of the arrangement 1 and entirely covers said arrangement 1. Said arrangement 1 is removably mounted on said fixed section 9, an actuating means 12 being attached to said fixed section 9 which is connected by means of a transmission means 13 to said rotating handle 5 of the arrangement 1.

Said actuating means 12, for instance, can be selected as a motor with a rotational or linear motion and similar. Further, said transmission means 13, for instance, can be selected as a gear transmission, a friction transmission, a leverage transmission, and similar.

Said transmission means 13 is intended to transfer motion from the actuating means 12 to the rotating handle 5 and via said rod 7 to said movable piston 6. If, for example, said transmission means 13 is selected as a gear transmission, then a driver is mounted on an axle of the actuating means 12 and a follower is mounted on the rotating handle 5 or it can be a part of the rotating handle 5 itself. Optionally, at least one additional gear can be arranged between the driver and the follower in order for the transmission ratio to be changed. A rotational motion of the actuating means 12 is transferred via said gear transmission 13 to the rotating handle 5 and thus, via the rod 7 onto the piston 6. In this instance, the piston rod 7 is formed with a threaded section which cooperates with the opposite threaded section in the form of a nut and formed in the movable piston 6, enabling thus for said piston 6 to move up and down depending on the rotation direction of the actuating means 12. In certain cases, if for instance a power failure or a breakdown occurs resulting in a standstill of the actuating means 12, the blood may still be sampled in a manner to rotate the rotating handle 5 manually.

A possibility is provided according to the present invention, that said actuating means 12 being connected by means of the transmission means 13 to said rotating handle 5 of the arrangement 1 is associated with the free end of said swivelling section 10. In such an instance, said gears of said gear transmission mesh by means of swivelling said section 10 in to the operating position when the actuating means 12 is located directly above the rotating handle 5.

The advantage of the blood sampling device according to the present invention is shown in an electro-mechanical actuator enabling setting of different patient related blood sampling profiles, such as a profile for an infant, a profile for a grown up person, a profile for an elderly. This means, that the electronic parts of the device according to the present invention takes care that the blood sampling rate matches each particular patient. Each profile may be set in advance, by means of different colours of the holder, or the device may comprise a separate switch for each of the profiles.

## Claims

1. A blood sampling device comprising a piston-cylinder arrangement (1) as an intermediate storage for diluted blood, said piston-cylinder arrangement (1) comprises a cylindrical housing (2) cooperating with a through-flow channel (4) and being closed by means of a rotating handle (5), and a piston (6) moving within said cylindrical housing (2) and connected to said rotating handle (5) said piston (6) actuated by means of an actuating means (12) via a transmission means (13), **characterized *in that*** it comprises a holder (8) formed of at least one fixed section (9) and at least one swivelling section (10) mutually connected by means of a swivel joint (11), said swivelling section (10) being formed in a manner that in the operating position for a blood sampling the free end thereof is located in the area directly blood sampling the free end thereof is located in the area directly above said rotating handle (5) of the arrangement (1) which is removably mounted on said fixed section (9) of the holder (8), the actuating means (12) being attached to said fixed section (9) or to said swivelling section (10), the actuating means (12) being connected by means of the transmission means (13) to said rotating handle (5) of the arrangement (1).

2. Device according to any of the preceding claims, ***characterized in that*** said actuating means (12) is selected as a rotational motion motor, linear motion motor and similar.

3. Device according to any of the preceding claims, ***characterized in that*** said transmission means (13) is selected as a gear transmission, a friction transmission, a leverage transmission, and similar.

4. Device according to any of the preceding claims, ***characterized in that*** a driver of the transmission means (13) is mounted on an axle of the actuating means (12) and a follower of the transmission means (13) is mounted on the rotating handle (5).

5. Device according to any of the preceding claims, ***characterized in that*** the follower of the transmission means (13) is a part of the rotating handle (5).

6. Device according to any of the preceding claims, ***characterized in that*** said gears of said gear transmission (13) mesh by means of swivelling said section (10) in to the operating position when the actuating means (12) is located directly above the rotating handle (5).

## Patentansprüche

1. Blutentnahmevorrichtung mit einer Kolben - Zylinder - Anordnung (1) als Zwischenspeicher für verdünntes Blut, wobei die besagte Kolben - Zylinder - Anordnung (1) ein zylindrisches Gehäuse (2), welches mit einem Durchflusskanal (4) verbunden ist und mittels eines Drehgriffs (5) verschlossen werden kann, und einen Kolben (6) umfasst, der sich in dem besagten zylindrischen Gehäuse (2) bewegt und mit dem besagten Drehgriff (5) verbunden ist, wobei der besagte Kolben (6) mithilfe eines Betätigungsmittels (12) über ein Übertragungsmittel (13) betätigt werden kann, ***dadurch gekennzeichnet, dass*** die Blutentnahmevorrichtung eine Halterung (8) umfasst, die wenigstens einen festen Abschnitt (9) und wenigstens einen schwenkbaren Abschnitt (10) aufweist, welche über ein Drehgelenk (11) miteinander verbunden sind, wobei der besagte schwenkbare Abschnitt (10) in einer Weise ausgebildet ist, dass sich dessen freies Ende in der Arbeitsposition zur Blutentnahme in dem Bereich direkt oberhalb des besagten Drehgriffs (5) der Anordnung (1) befindet, welcher abnehmbar an dem besagten befestigten Abschnitt (9) der Halterung (8) befestigt ist, wobei das Betätigungsmittel (12) an dem besagten festen Abschnitt (9) oder an dem besagten schwenkbaren Abschnitt (10) befestigt ist, und wobei das Betätigungsmittel (12) über das Übertragungsmittel (13) mit dem besagten Drehgriff (5) der Anordnung (1) verbunden ist.

2. Vorrichtung nach einem der vorhergehenden Ansprüche, ***dadurch gekennzeichnet, dass*** das besagte Betätigungsmittel (12) ein Drehbewegungsmotor, Linearbewegungsmotor oder ähnliches ist.

3. Vorrichtung nach einem der vorhergehenden Ansprüche, ***dadurch gekennzeichnet, dass*** das besagte Übertragungsmittel (13) als ein Zahnradgetriebe, ein Reibgetriebe, ein Hebelübersetzungsgetriebe oder ähnliches ausgebildet ist.

4. Vorrichtung nach einem der vorhergehenden Ansprüche, ***dadurch gekennzeichnet, dass*** ein Treiber des Übertragungsmittels (13) an einer Achse des Betätigungsmittels (12) befestigt ist und ein Mitnehmer des Übertragungsmittels (13) an dem Drehgriff (5) befestigt ist.

5. Vorrichtung nach einem der vorhergehenden Ansprüche, ***dadurch gekennzeichnet, dass*** der Mitnehmer des Übertragungsmittels (13) ein Teil des Drehgriffs (5) ist.

6. Vorrichtung nach einem der vorhergehenden Ansprüche, ***dadurch gekennzeichnet, dass*** die Zahnräder des Zahngetriebes durch Schwenken des besagten Abschnitts (10) in die Arbeitsposition ineinandergreifen, wenn sich das Betätigungsmittel (12) direkt oberhalb des Drehgriffs (5) befindet.

## Revendications

1. Dispositif de prélèvement sanguin comprenant un agencement piston-cylindre (1) comme dispositif de stockage intermédiaire pour du sang dilué, ledit agencement piston-cylindre (1) comprend un logement cylindrique (2) qui coopère avec un canal d'écoulement (4) et est fermé au moyen d'une poignée rotative (5), et un piston (6) se déplaçant à l'intérieur dudit logement cylindrique (2) et relié à ladite poignée rotative (5), ledit piston (6) étant actionné à l'aide d'un moyen d'actionnement (12) par l'intermédiaire d'un moyen de transmission (13), ***caractérisé par le fait qu'**il* comprend un support (8) formé d'au moins une section fixe (9) et d'au moins une section pivotante (10) reliées mutuellement à l'aide d'un joint pivotant (11), ladite section pivotante (10) étant formée d'une manière telle que, dans la position de fonctionnement pour un prélèvement sanguin, l'extrémité libre de celle-ci se situe dans la région directement au-dessus de ladite poignée rotative (5) de l'agencement (1) qui est monté de façon amovible sur ladite section fixe (9) du support (8), le moyen d'actionnement (12) étant fixé à ladite section fixe (9) ou à ladite section pivotante (10), le moyen d'actionnement (12) étant relié à l'aide du moyen de transmission (13) à ladite poignée rotative (5) de l'agencement (1).

2. Dispositif selon la revendication précédente, ***caractérisé par le fait que*** ledit moyen d'actionnement (12) est choisi sous la forme d'un moteur à mouvement rotatif, d'un moteur à mouvement linéaire et similaire.

3. Dispositif selon l'une quelconque des revendications précédentes, ***caractérisé par le fait que*** ledit moyen de transmission (13) est choisi sous la forme d'une transmission par engrenages, d'une transmission par frottement, d'une transmission par levier et similaire.

4. Dispositif selon l'une quelconque des revendications précédentes, ***caractérisé par le fait* qu'**un élément d'entraînement du moyen de transmission (13) est monté sur un axe du moyen d'actionnement (12) et un élément entraîné du moyen de transmission (13) est monté sur la poignée rotative (5).

5. Dispositif selon l'une quelconque des revendications précédentes, ***caractérisé par le fait que*** l'élément entraîné du moyen de transmission (13) fait partie de la poignée rotative (5).

6. Dispositif selon l'une quelconque des revendications précédentes, ***caractérisé par le fait que*** lesdits engrenages de ladite transmission par engrenages (13) s'engrènent à l'aide de ladite section pivotante (10) dans la position de fonctionnement lorsque le moyen d'actionnement (12) se situe directement au-dessus de la poignée rotative (5).
